Europäisches Patentamt

European Patent Office

Office européen des brevets

(11) Publication number: **0 173 494**
A2

## EUROPEAN PATENT APPLICATION

(21) Application number: 85305604.2

(22) Date of filing: 07.08.85

(51) Int. Cl.⁴: **C 12 N 15/00,** C 07 K 15/00, C 12 N 5/00, C 12 P 21/00

(30) Priority: 27.08.84 US 644473

(43) Date of publication of application: 05.03.86 Bulletin 86/10

(84) Designated Contracting States: DE FR GB SE

(71) Applicant: THE BOARD OF TRUSTEES OF THE LELAND STANFORD JUNIOR UNIVERSITY, Stanford, CA 94305 (US)

(72) Inventor: Morrison, Sherie L., 31 Claremont Road, Scarsdale New York, 10583 (US)
Inventor: Herzenberg, Leonard A., 876 Cedro Way, Stanford California 94305 (US)
Inventor: Oi, Vernon T., 316 Waverly, No. 3, Menlo Park California 94025 (US)

(74) Representative: Harrison, David Christopher et al, MEWBURN ELLIS & CO 2/3 Cursitor Street, London EC4A 1BQ (GB)

(54) Chimeric receptors by DNA splicing and expression.

(57) Chimeric multi-subunit receptors are provided involving a binding region resulting from the organization of two subunit chains and a non-binding region which may have physiological significance. Particularly, chimeric immunoglobulins are provided having variable regions from one species and constant regions from another species, by linking DNA sequences encoding for the variable regions of the light and heavy chains from one species to the constant regions of the light and heavy chains respectively from a different species. Introduction of the resulting genes into mammalian host cells under conditions for expressions provides for production of chimeric immunoglobulins having the specificity of the variable region derived from a first species and the physiological functions of the constant region from a different species.

# CHIMERIC RECEPTORS BY DNA SPLICING AND EXPRESSION

Naturally occurring receptors, such as immunoglobulins, enzymes, and membrane proteins have seen an extraordinary expansion in commercial applications over the last decade. With the advent of monoclonal antibodies, the usefulness of immunoglobulins has been greatly expanded and in many situations has greatly extended prior uses employing polyclonal antibodies. However, in many applications, the use of monoclonal antibodies is severely restricted where the monoclonal antibodies are to be used in a biological environment. Since, for the most part, monoclonal antibodies are produced in rodents, e.g., mice, the monoclonal antibodies are immunogenic to other species.

While the constant regions of immunoglobulins are not involved in ligand binding, the constant regions do have a number of specific functions, such as complement binding, immunogenicity, cell receptor binding, and the like. There will, therefore, be situations where it will be desirable to have constant regions which bind to cells or proteins from a particular species having binding regions for a particular ligand.

Kwan et al., J. Exp. Mod. (1981) 153:1366-1370 and Clarke et al., Nucl. Acids Res. (1982) 10:7731-7749 describe $V_H$ and $V_\kappa$ exons from the mouse phosphocholine-binding antibody producing S107 myeloma cell line. Oi et al., Proc. Natl. Acad. Sci. USA (1983) 80:825-829, report that the mouse light chain gene is not expressed efficiently in a rat myeloma cell.

In this invention chimeric multi-subunit receptors are provided, where each of the subunits is an expression product of a fused gene. Each fused gene comprises a DNA sequence from one host species encoding the region involved with ligand binding joined to a DNA sequence from a different host species encoding a "constant" region providing a structural framework and biological properties. Introduction of the fused genes into an appropriate eukaryotic host cell under conditions for expression and processing provides for a functional assembled multi-subunit receptor product.

In the accompanying drawings:

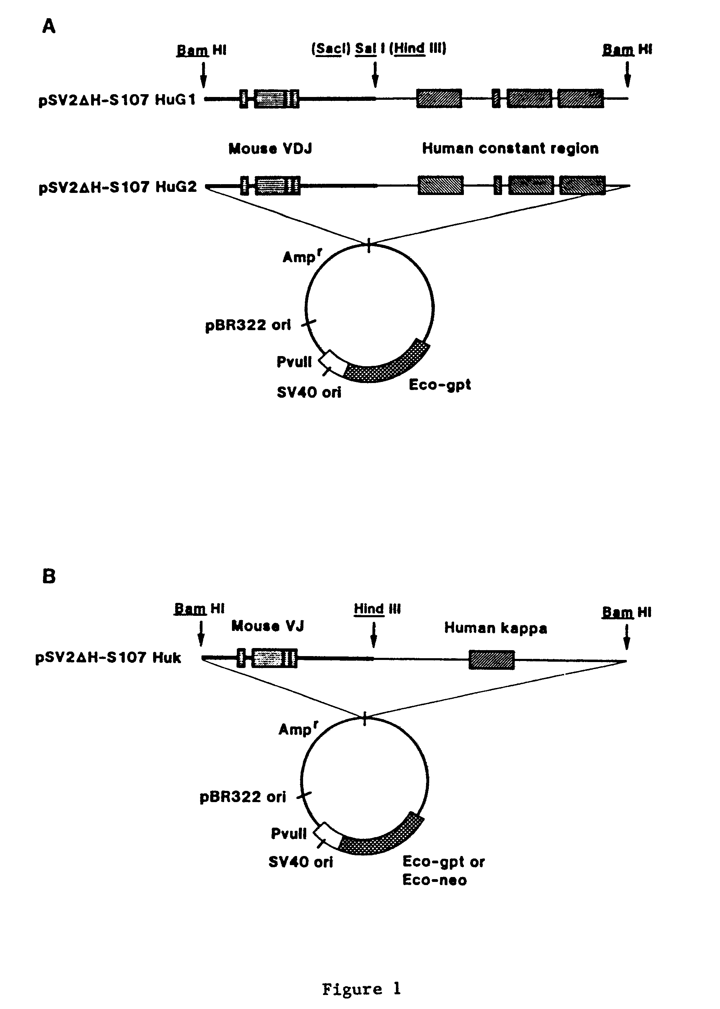

Figure 1 is a schematic diagram of (a) the chimeric mouse:human heavy chain gene vector; and (b) the chimeric light chain vector.

## DESCRIPTION OF THE SPECIFIC EMBODIMENTS

Novel methods and compositions are provided for production of polypeptide products having specific binding affinities for a predetermined ligand and predetermined biological, particularly physiological, properties, not naturally associated with the binding region peptide sequences. Particularly, multi-subunit chimeric receptors are provided which result from fused genes having the portion of the polypeptide involved with binding of a predetermined ligand having an amino acid sequence substantially ($>90\%$ conserved) the same as an amino acid sequence having the same function from one host species, while the portion involved with providing structural stability, as well as other biological functions, being analogously derived from a different host species. At least two fused genes are involved, which genes are introduced into an appropriate eukaryotic host under conditions for expression and processing, whereby the fused genes are expressed and the resulting subunits bound together, resulting in an assembled chimeric receptor.

The receptors prepared in accordance with the subject invention will be multi-subunit, where the units are held together either by non-covalent binding or a combination of non-covalent and covalent binding, particularly disulfide linkages through cysteine, and having at least one binding, usually at least two binding sites and not more than about ten binding sites. Receptors of interest include both B-cell and T-cell receptors, more particularly, immunoglobulins, such as IgM, IgG, IgA, IgD and IgE, as well as the various subtypes of the individual groups. The light chain may be $\kappa$, or $\lambda$. The heavy chains are referred to as $\mu$, $\gamma$, $\delta$, $\alpha$ and $\epsilon$.

In discussing the two regions of each subunit, the two regions will be referred to as "variable" and "constant" by analogy to immunoglobulins. The variable region is the region involved with ligand binding and, therefore, will vary in conformation and amino acid sequence depending upon the ligand. The region will usually be composed of a plurality of domains, involving a domain having as its primary function binding to the ligand (V) and a domain associated with joining the V region to the constant region, the hinge region (J). There may also be a hypervariable domain intermediate V and J and joining the V and J domains, the diversity domain (D). These domains are related to exons observed in the genes encoding for the variable regions.

The constant region will not be associated with ligand binding and will be relatively limited in the variations in its conformation and amino acid sequence within any one species within a class, each class generally having from 1 to 4 subclasses. Each constant region is specific for a species. Within the classes there will be allotypes, usually not exceeding about 30 allotypes.

The variable region of the immunoglobulins will be derived from a convenient mammalian source, which may be a rodent, e.g., mouse or rat, rabbit, or other vertebrate, mammalian or otherwise, capable of producing immunoglobulins. The constant region of the immunoglobulin, as well as the J region for IgM, will be derived from a vertebrate source different from the source of the variable region, particularly a mammalian source, more particularly primate or domestic animal, e.g., bovine, porcine, equine, canine, feline, or the like, and particularly, humans. Thus, the immunogenic portion of the receptor will normally be chosen in accordance with the purpose of the receptor. For example, where the receptor is to be introduced into the host, the constant or immunogenic portion will be selected so as to minimize the immune response of the host. Where the receptor is to bind to particular cell membrane surface receptors, the constant region will be chosen in accordance with the host of the receptor recognition site.

The fused gene derived from the two host sources will be prepared by joining the 5' end of a sequence encoding the constant region in reading frame to the 3'-end of a sequence encoding the variable region. (In referring to 5' or 3' for a double strand, it is intended the direction of transcription with 5' being upstream from 3'.) With immunoglobulins, two fused genes will be prepared, one for the light chain and one for the heavy chain. With T-cell receptors, the two fused genes will be for each of the two chains involved in the formation of the T-cell receptor. The DNA sequences employed for preparation of the fused gene, may be derived from a variety of sources. These sources include genomic DNA, cDNA, synthetic DNA, and combinations thereof. The genomic DNA may or may not include naturally occurring introns.

The DNA obtained from natural sources, namely the genomic DNA or cDNA, may be obtained in a variety of ways. Host cells coding for the desired sequence may be isolated, the genomic DNA fragmented, conveniently by one or more restriction endonucleases, and the resulting fragments cloned and screened with a probe for the presence of the DNA sequence coding for the polypeptide sequence of interest. For the variable region, the rearranged germline heavy chain DNA will include V, D, and J regions, including the leader sequence, which may be subsequently removed as well as any introns. The rearranged germline light chain coding DNA will include the V and J regions including the leader sequence, as well as any introns which may be subsequently removed. The particular source of the exons defining the domains and the manner of splicing, where introns are present, is not germane to this invention. Once the cloned fragment has been identified which contains the desired DNA sequence, this fragment may be further manipulated to remove superfluous DNA, modify one or both termini, remove all or a portion of intervening sequences, (introns) or the like.

In providing a fragment encoding the variable region, it will usually be desirable to include all or a portion of the intron downstream from the J region, particularly where the variable region is derived from the host in which the fused gene is to be expressed. Where the intron is retained, it will be necessary that there be functional splice acceptor and donor sequences at the intron termini. The intron between the J (hinge region) and the constant region of the fused gene may be primarily the intron sequence associated with (1) the constant region, (2) the J domain, or (3) portions of each. The last may be a matter of convenience where there is a convenient restriction site in the introns from the two sources. In some instances, all or a portion of the intron may be modified by deletion, nucleotide substitution(s) or insertion, to enhance ease of manipulation, expression, or the like. Since in many cases the variable region will be synergenic with the host cells employed for expression, preferably all or at least about 80% of the intron sequence will be the naturally occurring intron sequence associated with the J domain. In some instances it will be necessary to provide adapters to join the intron or truncated intron to the constant region. By cleaving within the intron, the variable region will be separated from its natural constant region.

Alternatively, it may be desirable to have the fused gene free of the intron between the variable and constant regions. Thus, the 3' terminus will be at or in the hinge domain. Normally all or a portion of the hinge domain will be associated with the host providing the variable region. By restriction enzyme analysis or sequencing of the hinge domain, one can select for a particular site for the 3' terminus of the variable region.

Alternatively, one can use an exonuclease and by employing varying periods of digestion, one can provide for varying 3'-termini, which can then be used for linking to the constant region and selection made for a functional product in a variety of ways. For example, where joining of the variable region to the constant region results in a unique restriction site, the fused DNA fragments may be screened for the presence of the restriction site. Alternatively, it may be found desirable to include an adapter or linker to join the variable region to the constant region, where the adapter or linker may have the same or substantially the same sequence, usually at least substantially the same sequence, of the DNA sequence of the two fragments adjacent the juncture. The adapter or linker will be selected so as to provide for the two sequences to be in common reading frame. Furthermore, by employing adapters, one could add an additional degree of variability in the binding affinity of the chimeric receptor, by providing for the expression of different amino acids in the hinge domain.

The joining of the various fragments is performed in accordance with conventional techniques, employing blunt-ended or staggered-ended termini for ligation, restriction enzyme digestion to provide for appropriate termini, filling in of cohesive ends as appropriate, alkaline phosphatase treatment to avoid undesirable joining, and ligation with appropriate ligases.

For cDNA, the cDNA may be cloned and the resulting clone screened with an appropriate probe for cDNA coding for the desired variable or constant region. Once the desired clone has been isolated, the cDNA may be manipulated in substantially the same manner as the genomic DNA. However, with cDNA there will be no introns or intervening sequences. The cDNA is cleaved at or near the juncture of the variable region with the constant region so that the variable region is separated from the constant region and the desired region retained. Where a convenient restriction site exists, the cDNA may be digested to provide for a fragment having the appropriate terminus. The restriction site may provide a satisfactory site or be extended with an adapter. Alternatively, primer repair may be employed, where, for the variable region a complementary sequence to the site of cleavage and successive nucleotides in the 3' direction of the complementary sequence is hybridized to the sense strand of the cDNA and the nonsense strand replicated beginning with the primer and removal of the single-stranded DNA of the sense strand 3' from the primer. The reverse is true is true for the constant region. Other techniques may also suggest themselves. Once the fragment has been obtained having the predetermined 3' or 5' terminus, as appropriate, it may then be employed for joining to the other region.

Finally, one or both of the regions may be synthesized and cloned for use in preparing the fused gene. For the most part, the same or substantially the same constant region can be repetitively used, so that a library of constant regions may be established which can be selected for joining to variable regions. Thus, the constant regions would have an appropriate 5' terminus for joining directly or through an adapter to a variable region.

In order for expression of the fused gene, it will be necessary to have transcriptional and translational signals recognized by an appropriate eukaryotic host. For the most part, desirable eukaryotic hosts will be mammalian cells capable of culture in vitro, particularly leukocytes, more particularly myeloma cells, or other transformed or oncogenic lymphocyte, e.g., EBV transformed cells. Alternatively, non-mammalian cells may be employed, such as fungi, e.g., yeast, filamentous fungi, or the like.

The DNA sequence coding for the variable region may be obtained in association with the promoter region from genomic DNA. To the extent that the host cells recognize the transcriptional regulatory and translational initiation signals associated with the variable region, then the region 5' of the variable region coding sequence may be retained with the variable region coding sequence and employed for transcriptional and translational initiation regulation.

The contiguous non-coding region 5' to the variable region will normally include those sequences involved with initiation of transcription and translation, such as the TATA box, capping sequence, CAAT sequence, and the like. Usually the 5'-non-coding sequence will be at least 150bp, more usually at least 200bp, usually not exceeding about 2kbp, more usually not exceeding about 1kbp.

The non-coding region 3' to the constant region may be retained for its transcriptional termination regulatory sequences, such as termination and polyadenylation. Thus, by retaining the 3'-region naturally contiguous to the DNA sequence coding for the constant region, the transcriptional termination signals may be provided for the fused gene. Where the transcriptional termination signals are not satisfactorily functional in the expression host cell, then a 3' region functional in the host cell may be substituted. Conveniently, the non-coding 3' region may be obtained from a non-coding contiguous 3' region of a constant region from the expression host. The 3'-non-coding region may be joined to the constant region by any of the means described previously for manipulation and ligation of DNA fragments. This region could then be used as a building block in preparing the fused gene.

The fused gene for the most part may be depicted by the following formula:

$$\text{TIR-(LS)}_e\text{-V}_c\text{-(Vi)}_a\text{-(D)}_b\text{-(Di)}_c\text{-J-(I)}_d\text{-C-TTR}$$

wherein:

TIR intends the transcriptional regulatory and translational initiation region, is generally of at least about 150bp and not more than about 2kbp, which may be in whole or in part the sequence naturally joined to the $V_c$ coding region;

LS refers to a DNA sequence encoding for a leader sequence and processing signal functional in the expression host for secretion and processing for removal of the sequence;

$V_c$ is an exon coding for the variable domain in reading frame with LS, when LS is present;

(e) is 0 or 1;

$D_c$ is an exon coding for the diversity domain and is present for the heavy chain (b=1) and is absent for the light chain (b=0);

J is an exon coding for the hinge region;

$V_i$ and $D_i$ are introns naturally associated with the letter indicated exons having functional donor and acceptor splicing sites;

a, b and c are the same or different and are 0 or 1, wherein when b is 0, c is 0;

I is an intron which may be naturally contiguous to the J exon or naturally contiguous to the C exon or a combination of fragments from both, or a fragment thereof, desirably including an enhancer sequence functional in said expression host, or I may be foreign in whole or in part to the J and C exons;

d is 0 or 1;

c is the constant domain and may code for μ, γ, δ, α and ε chain, preferably μ or γ, usually including at least 80% of the constant region sequence, and may be the same as or a modified naturally occurring allotype; and

TTR is the transcriptional termination region providing for transcriptional termination and polyadenylation which may be naturally associated with C or may be joined to C, being functional in the expression host; usually being at least about 100bp and may be 1kbp or more.

The constructs for each of the different subunits may be joined together to form a single DNA segment or may be maintained as separate segments, by themselves or in conjunction with vectors.

The subunit constructs may be introduced into a cell by transformation in conjunction with a gene allowing for selection where the construct will become integrated into the host genome. Usually the construct will be part of a vector having a replication system recognized by the host cell.

A large number of vectors are available or can be readily prepared which provide for replication and expression in a host, either by maintenance as an extrachromosomal element or by integration into the host genome. For a mammalian host, a wide variety of vectors are known based on viral replication systems, such as Simian virus, bovine papilloma virus, adenovirus and the like. These vectors can be used as expression vectors where transcriptional and translational initiation and termination signals are present and one or more restriction sites are available for insertion of a structural gene. In addition, the vectors normally have one or more markers which allow for selection of host cells which contain the expression vector. The marker may provide for prototrophy to an auxotrophic host, biocide resistance, e.g., antibiotics, such as G418, or heavy metals, such as copper, or the like. If desired, expression vectors can be prepared by joining the various components, such as the replication system, markers, and transcriptional and translational regulatory initiation and termination signals in conjunction with the fused gene. Frequently, a vector will include a prokaryotic replication system, which allows for cloning after each of the manipulative steps to provide for purification and expansion of the desired DNA sequence.

A wide variety of transcriptional and translational regulatory sequences may be employed, depending upon the nature of the host. The transcriptional and translational regulatory signals may be derived from viral sources, such as adenovirus, bovine papilloma virus, Simian virus, or the like, where the regulatory signals are associated with a particular gene which has a high level of expression. Alternatively, promoters from mammalian expression products, such as actin, collagen, myosin, etc., may be employed. Transcriptional initiation regulatory signals may be selected which allow for repression or activation, so that expression of the fused genes can be modulated. Of interest are regulatory signals which are temperature-sensitive so that by varying the temperature, expression can be repressed or initiated, or are subject to chemical regulation, e.g., metabolite.

Once the vector or DNA sequence containing the fused gene has been prepared for expression, the DNA construct may be introduced into an appropriate host. Various techniques may be employed, such as protoplast fusion, calcium phosphate-precipitation, or other conventional technique. After the fusion, the cells are grown in a selective medium, where untransformed cells are killed, leaving only cells transformed with the DNA construct. Expression of the fused gene results in assembly to form the receptor.

The host cells will for the most part be immortalized cells, particularly myeloma or lymphoma cells. These cells may be grown in an appropriate nutrient medium in culture flasks or injected into a synergenic host, e.g., mouse or rat, or immunodeficient host or host site, e.g., nude mouse or hamster pouch. Particularly, the cells may be introduced into the abdominal cavity for production of ascites fluid and harvesting of the chimeric receptor. Alternatively, the cells may be injected subcutaneously and the antibodies harvested from the blood of the host. The cells may be used in the same manner as hybridoma cells. See Diamond et al., N. Eng. J. Med. (1981) 304:1344 and Kennatt, McKearn and Bechtol (eds.), Monoclonal Antibodies: Hybridomas--A New Dimension in

<u>Biologic Analysis</u>, Plenum, 1980, which are incorporated herein by reference.

Where a leader is present with a processing signal for secretion and selective cleavage of the leader sequence, the resulting assembled receptor will be secreted into the nutrient medium of the transformed cells and may be harvested. Where secretion does not occur, after sufficient time for the receptor to be expressed in reasonable amounts, the cells may be killed, lysed, and the receptors isolated and purified. Where transcriptional initiation can be modulated, the cells may be grown to high density under non-permissive conditions, followed by growth under permissive conditions where the receptor is expressed.

The receptors may be naturally glycosylated, unnaturally glycosylated or be free of glycosyl groups, depending on the host, conditions of cellular growth and subsequent treatment. Where a mammalian host cell is employed for expression, usually natural glycosylation will occur. Glycosylation can be prevented by an appropriate inhibitor, e.g., tunicamycin. Alternatively, glycosyl groups may be removed by hydrolysis, e.g., enzymatic hydrolysis using hydrolases. In expression hosts other than mammalian cells, unglycosylated or unnatural glycosylated receptors may be obtained.

The receptor may be isolated and purified in accordance with conventional conditions, such as extraction, precipitation, chromatography, affinity chromatography, electrophoresis, or the like. By employing antibodies specific for the constant region(s), affinity chromatography will allow for concentration and purification of the chimeric receptor.

The chimeric receptors can be used in the same manner as other receptors for binding to specific ligands in diagnostic assays, affinity chromatography, or the like. In addition, because of a substantially reduced immunogenicity of the chimeric receptors, the chimeric receptors can find use in therapy, for passive immunization, for in vivo imaging, for specific treatment of diseased cells, or the like. For in vivo imaging, the chimeric antibody will normally be conjugated to a radionuclide, e.g., technicium, rhenium, or the like. For biocidal activity, the antibody may be joined to the A-portion of toxins, liposomes containing biocidal reagents, radionuclides, or other biocidal agent. Alternatively, the antibodies can be used in combination with the host immune system, e.g., complement, due to the presence of the native constant region. In vitro, the subject chimeric antibodies can be used in conjunction with complement to remove particular cells from a mixture of cells, where the target cells have a ligand complementary to the binding site of the chimeric antibody.

The following examples are offered by way of illustration and not by way of limitation.

EXPERIMENTAL

Materials and Methods

Chimeric genes

The cloned S107 variable region (heavy) (VH) and S107 $V_\kappa$ variable region (light, kappa) genes were obtained from Dr. Matthew Scharff (Dept. of Cell Biology, Albert Einstein College of Medicine, Bronx, NY 10641). The S107 VH gene was spliced to human IgG1 and IgG2 constant region genes using SalI linkers as shown in Figure 1. Both constructs were inserted into the vector pSV2ΔH-gpt (Oi et al., Proc. Natl. Acad. Sci. USA (1983) 80:825-829; Mulligan and Berg, Science (1980) 209:1422-1427). The S107 $V_\kappa$ gene was spliced to the human $\kappa$ gene at a unique HindIII site located in the large intron between the $J_\kappa$ and $C_\kappa$ exons as shown in Figure 2. This chimeric light chain gene construct was inserted into both pSV2 ΔH-gpt and pSV2-neo plasmid vectors (Mulligan and Berg, <u>Proc. Natl. Acad. Sci. USA</u> (1981) <u>78</u>:2072-2076).

Transfection

Protoplast fusion and calcium-phosphate ($CaPO_4$) precipitation techniques (Oi <u>et al</u>., (1983), <u>supra</u>; Sandri-Goldin <u>et al</u>., <u>Mol. Cell. Biol.</u> (1981) <u>1</u>:743-752; Chu and Sharp, <u>Gene</u> (1980) <u>13</u>:197-202) were used to transfect these chimeric immunoglobulin genes into the J558L myeloma cell line (a lambda (λ) light chain producing mouse myeloma cell line) and the non-immunoglobulin-producing An derivative of the P3 myeloma cell line. Mycophenolic acid (Gibco Laboratories, Santa Clara, CA 95050) was used for selection of cells transfected with pSV2 ΔH-gpt vectors as described previously (Oi <u>et al</u>., (1983), <u>supra</u>; Ochi <u>et al</u>., <u>Proc. Natl. Acad. Sci. USA</u> (1983) <u>80</u>:6351-6355). G418 (Gibco Laboratories)·at 1.0mg/ml was used for selection of cells transfected with pSV2-neo vectors (Mulligan and Berg (1980), <u>supra</u>).

When both light and heavy chimeric genes were transfected into the J558L cell line using protoplast fusion techniques, light and heavy chimeric immunoglobulin genes were transfected sequentially using G418 selection for the chimeric light chain gene vector and mycophenolic acid for the chimeric heavy chain gene vector. The protoplast fusion transfection procedure used was as described previously (Oi <u>et al</u>., (1983), <u>supra</u>).

Transfection using the CaPO4 precipitation procedure was done by mixing 40 µg of both chimeric light and chimeric heavy chain pSV2 ΔH-gpt vectors and transfecting a total of 80 µg of plasmid DNA into $5 \times 10^6$ cells. Mycophenolic acid was used to select for transformed cell lines as described previously (Oi <u>et al</u>., (1983), <u>supra</u>).

0173494

Antigen-binding

Phosphocholine (PC) binding of antibody secreted into the culture supernates of transfected cell lines was analyzed using a solid-phase radioimmunoassay described previously (Oi and Herzenberg, Mol. Immunol. (1979) 16:1005-1017). PC-binding antibodies in biosynthetically-labeled culture supernates and cell lysates of transfected cell lines also were analyzed by binding the biosynthetically-labeled antibody to PC-coupled to Sepharose 4B (Pharmacia Five Chemicals, Piscataway, N.J.) and then eluting the bound antibody with PC-hapten. The bound and eluted antibody was examined by SDS-polyacrylamide gel electrophoresis (SDS-PAGE). Biosynthetic-labeling procedures were done as described previously (Oi et al., J. Exp. Med. (1980) 151:1260-1274).

Idiotype analysis

Three hybridoma anti-idiotope antibodies, obtained from Dr. Matthew Scharff (Dept. of Cell Biology, Albert Einstein College of Medicine, Bronx, NY), were used to analyze the variable heavy-variable light (VH-VL) domain structure of the chimeric human anti-PC antibodies. These antibodies, recognizing three independent idiotopes, were used to immunoprecipitate biosynthetically-labeled material eluted with PC from the PC-Sepharose 4B matrix. Immunoprecipitates were analyzed by SDS-PAGE.

Immunoglobulin Chain composition

Monoclonal anti-human IgG and anti-human κ antibodies (Becton-Dickinson Monoclonal Center, Mountain View, CA) were used to immunoprecipitate biosynthetically-labeled chimeric human anti-PC antibodies for analyses using two-dimensional non-equilibrium pH gradient polyacrylamide gel electrophoresis (NEPHGE) (Oi and Herzenberg (1979), supra). PC-coupled to Sepharose 4B also was used for immunoprecipitations.

## Immunoglobulin Heavy Chain Glycosylation

Tunicamycin (Calbiochem-Behring, San Diego, CA) was used to inhibit asparagine-linked glycosylation of biosynthetically-labeled antibody from mouse cell lines producing mouse:human chimeric immunoglobulins (Oi et al. (1980), supra). PC-binding antibody from tunicamycin-treated cells was analyzed by SDS-PAGE. Procedures used for tunicamycin treatment were as described previously (Oi et al., (1980), supra).

## Chimeric mouse:human antibody production in mice

Transformed J558L cells producing chimeric mouse:human antibody were injected subcutaneously into Balb/c mice ($10^6$ cells/mouse). Sera from tumor-bearing mice were analyzed for human anti-PC antibody by a solid-phase radioimmunoassay described previously (Oi and Herzenberg (1979), supra) and by immunoelectrophoresis using a polyclonal anti-human antiserum.

## Results

Expression of chimeric mouse V:human C region genes in transfected mouse myeloma cells J558L and the non-immunoglobulin-producing P3 myeloma cell lines was obtained. When both light chain and heavy chain chimeric genes were transfected into the same cell, tetrameric (H2L2) antigen-binding antibodies were obtained. Autoradiograms of two-dimensional NEPHGE analyses of the chain composition of biosynthesized and secreted antibody molecules bound and eluted from phosphocholine-Sepharose showed the formation of mixed molecules, including the endogenously produced J558L λ light chain. Each polypeptide chain had the expected charge and relative molecular weight. Identical two-dimensional gel analyses results were obtained with immunoprecipitates with monoclonal anti-human κ and IgG antibodies. Similar results were obtained from immunoprecipitates of human $IgG_2$(κ) antibodies produced by transfected P3 cell lines. Since the non-immunoglobulin-producing parental P3 cell line does not produce endogenous immunoglobulin polypeptide chains, as expected only the chimeric mouse:human heavy and light chains are seen on the autoradiograms.

Phosphocholine-binding by the chimeric antibody produced in the J558L cell line was the result from specific association of the chimeric immunoglobulin light and heavy polypeptide chains, i.e., the VH and Vκ domains of the S107 myeloma protein connected to human constant region polypeptides. This was determined by measuring PC-binding by immunoglobulins produced by J558L cells transfected with the mouse:human chimeric heavy chain gene. PC-binding was never observed from antibody secreted by transfected J558L cells expressing only the chimeric heavy chain and the endogenous J558L λ light chain (data not shown). Chimeric antibodies produced in the transfected non-immunoglobulin-producing P3 cell line also were shown to bind PC-Sepharose. In view of the low binding affinity of the parental mouse S107 antibody, analyses of appropriate polypeptide folding of mouse VH and Vκ domains in the novel environment of human constant regions polypeptide chains were done by determining the presence of idiotypes known to occur on the parental S107 PC-binding antibody molecule. Three monoclonal anti-idiotope antibodies, each recognizing a distinct epitope on the light and heavy variable region domains and an epitope defined by the presence of both light and heavy variable region domains, were found to react with the mouse:human chimeric anti-PC antibodies. This strongly supports the fact that the mouse S107 antigen-binding domains have folded into their intended structures.

Glycosylation of the mouse:human chimeric antibodies in mouse myeloma cells was analyzed by measuring the relative molecular weight ($Mr$) of antibodies biosynthesized in the presence and absence of tunicamycin, a known antibiotic inhibitor of asparagine-linked glycosylation. Autoradiograms of SDS-PAGE analysis of the chimeric heavy and light chains produced in mouse myeloma cells in the presence or absence of tunicamycin showed the lower relative $Mr$ of the heavy chain synthesized in the presence of tunicamycin as expected if a single N-linked carbohydrate was absent from the polypeptide chain. From this data it is concluded that the mouse myeloma cell appropriately glycosylates the human heavy chain.

When transfected J558L cells producing the human IgG2($\kappa$) chimeric anti-PC antibody was grown as a subcutaneous tumor in Balb/c mice, analysis of the sera of these mice showed significant human IgG2($\kappa$) anti-PC binding antibody production of the chimeric antibody molecules by radioimmunoassay. Polyclonal anti-human antiserum demonstrated the presence of significant quantities of human immunoglobulin in the sera. Based on comparison with prior experience with mouse hybridoma-antibody production in mice, the amounts of immunoglobulin visualized by immunoelectrophoresis analysis of mice bearing tumors of the transfected J558L cell line was similar to the lower levels of production seen with other mouse hybridoma tumor cell lines.

Analysis showed that fewer than about ten percent of the transfected cell lines produced both chimeric heavy and light chain polypeptides. Among transformants generated by protoplast fusion, both gpt and neo biochemical markers were expressed at expected frequencies. However, chimeric light chain expression was infrequent. In co-transfection experiments using the $CaPO_4$ precipitation protocol, the same phenomenon was observed.

Based on prior experience with coexpression of gene products in transformed cell lines, it appears that the appropriate transcriptional or translational controlling elements are absent in either the chimeric light chain gene construct or in the mouse myeloma cell lines used. The mouse Vκ gene promoter is coupled to the presumed human intronic DNA sequences that are homologous to the known mouse intronic controlling element (ICE) or immunoglobulin "enhancer" element (Morrison and Oi, Ann. Rev. Immunol. (1984) 7:239-256). The chimeric mouse:human heavy chain gene is not constructed in this manner and, in contrast, is expressed efficiently. The mouse heavy chain intronic controlling element (ICE) sequences are included and human sequences excluded in this construct. The basis for the low level of expression of the light chain is subject to speculation.

It is evident from the above results that chimeric receptors, as illustrated by immunoglobulins, can be produced where the variable regions may be obtained from one host source and the constant regions obtained from another host source. Where the immunoglobulins are to be used in vivo, this can provide for numerous advantages, such as reduced immunogenicity, a lower catabolism, and the ability to fulfill biological effector functions associated with the constant regions. Furthermore, now that it is shown that chimeric receptors can be produced, there is the opportunity to prepare chimeric receptors with modification of J and D regions so as to modify binding specificity.

Although the foregoing invention has been described in some detail by way of illustration and example for purposes of clarity of understanding, it

21 0173494

will be obvious that certain changes and modifications may be practiced.

CLAIMS:

1. A multi-subunit chimeric receptor with at least one binding site and having two subunits which are bound together and define a predetermined ligand binding site, wherein each of said two subunits consists of a variable region which participates in defining said binding site and binding to said ligand and a non-binding region (constant region) which participates in maintaining the structure of said receptor, wherein each of said variable regions has an amino acid sequence having substantially the same amino acid sequence of a variable region of a receptor from a first mammalian source and each of said constant regions has an amino acid sequence having substantially the same amino acid sequence of a constant region from a different second mammalian source.

2. A multi-subunit chimeric receptor according to Claim 1, wherein said receptor is an immunoglobulin.

3. A multi-subunit chimeric receptor according to Claim 1 or Claim 2, joined to a label providing a detectable signal.

4. A DNA construct for expression of a chimeric polypeptide which is a subunit of a multi-unit receptor comprising:

a 5'-terminus proximal first DNA sequence coding for a polypeptide substantially the same as the N-terminal variable binding region of a receptor from a first mammalian host; and

a 3'-terminus proximal second DNA sequence joined directly or through an intron in reading frame at its 5' end to the 3' end of said first DNA sequence, coding for a polypeptide substantially the same as the non-binding region (constant region) from a different second mammalian host.

5. A DNA construct according to Claim 4, having transcriptional initiation and termination regulatory sequences recognized by a mammalian host in regulatory relationship with said first and second DNA sequences.

6. A DNA construct according to Claim 4 or Claim 5, wherein said first and second DNA sequences code for an immunoglobulin light chain.

7. A DNA construct according to Claim 4 or Claim 5, wherein said first and second DNA sequences code for an immunoglobulin heavy chain.

8. A mammalian cell having first and second DNA constructs for expression of different first and second subunits of a multi-unit receptor, said first and second subunits when bound together defining a binding site for a predetermined ligand;
    each of said first and second constructs comprising:
    a 5'-terminus proximal first DNA sequence coding for a polypeptide substantially the same as the N-terminal variable binding region of a receptor from a first mammalian host; and
    a 3'-terminus proximal second DNA sequence joined directly or through an intron in reading frame at its 5' end to the 3' end of said first DNA sequence coding for a polypeptide substantially the same as the non-binding region (constant region) from a different second mammalian host;
    and transcriptional and translational regulatory initiation and termination sequences functional in said mammalian cell and in regulatory relationship with said first and second DNA constructs.

9. A mammalian cell according to Claim 8, wherein said first DNA construct encodes an immunoglobulin light chain and second second DNA construct encodes an immunoglobulin heavy chain.

0173494

10. A method for producing a chimeric receptor which comprises:

growing mammalian cells according to Claim 8 or Claim 9 in a nutrient medium, whereby said DNA constructs are expressed and said subunits are intracellularly bound together to form a receptor; and

isolating said receptor.

11. A method according to Claim 10, wherein said cell is a mouse myeloma cell.

12. A method according to Claim 11, wherein said mouse myeloma cell is grown in the peritoneal cavity of a mouse resulting in the production of ascites fluid.

Figure 1